Europäisches Patentamt

European Patent Office

Office européen des brevets

(11) Publication number: **0 380 213**
**A2**

# EUROPEAN PATENT APPLICATION

(21) Application number: **90300313.5**

(22) Date of filing: **11.01.90**

(51) Int. Cl.⁵: **C07C 51/41, B01J 23/44,**
**C07C 53/126, C07C 53/128,**
**C07C 57/12, C07C 61/00,**
**C07C 63/08**

(30) Priority: **27.01.89 US 303499**

(43) Date of publication of application:
**01.08.90 Bulletin 90/31**

(84) Designated Contracting States:
**AT BE CH DE DK ES FR GB GR IT LI LU NL SE**

(71) Applicant: **MOONEY CHEMICALS, INC.**
**2301 Scranton Road**
**Cleveland Ohio 44113(US)**

(72) Inventor: **Nappier, Thomas E.**
**2338 Augustine Drive Parma**
**Ohio 44134(US)**
Inventor: **Tinker, H. Burnham**
**2889 Manchester Road Shaker Heights**
**Ohio 44122(US)**

(74) Representative: **Lambert, Hugh Richmond et
al**
**D. YOUNG & CO. 10 Staple Inn**
**London, WC1V 7RD(GB)**

(54) **Precious metal salt solutions and precious metal catalysts.**

(57) According to the disclosure, solutions of precious metal salts of organic carboxylic acids are prepared by reacting an organic carboxylic acid with a hydrated precious metal oxide in an organic solvent for both the acid and the carboxylate salt. The process results in high purity solutions which can be used as such as very effective precious metal catalysts. Alternatively, the precious metal carboxylates can be recovered and converted into oxides or elemental metal products by decomposition or reduction and giving rise to products having enhanced surface area and small particle clusters, and also useful as very effective precious metal catalysts.

EP 0 380 213 A2

## PRECIOUS METAL SALT SOLUTIONS AND PRECIOUS METAL CATALYSTS

The present invention relates to a method for preparing solutions of precious metal salts of organic carboxylic acids, to the solutions thus obtained, and to precious metal catalysts prepared from such solutions.

Many types and mixtures of metal salts and soaps of natural or synthetic organic acids, particularly carboxylic acids, have been suggested and commercially offered over several decades. These have been used to supply metals in forms which are soluble in organic liquids, particularly in various hydrocarbon oils and solvents, to form solutions having various desired properties and uses. For example, such metal salts have found uses as catalysts and as fuel and lubricant additives. Metal salts of carboxylic acids also are useful as stabilizers for various polymers including polyvinyl chloride-type plastics, and in the area of drying catalysts for paints, varnishes and other coating compositions.

The metal salts of carboxylic acids which have been described in the prior art include salts formed with a variety of metals including noble and precious metals such as silver, gold, platinum, palladium, osmium, rhodium, iridium, ruthenium, etc. Palladium, its oxides and salts, including organic as well as inorganic salts, have been widely used as catalysts for hydrogenation reactions and in oxidation reactions such as for the preparation of aldehydes, ketones, etc. by the catalytic liquid phase oxidation of hydrocarbons. Additionally, solutions containing precious metals in solvents such as hydrocarbon solvents are particularly desirable. Solutions of precious metal salts are of interest as catalysts for homogeneous solution polymerization of, for example, unsaturated aliphatic hydrocarbons such as ethylene, propylene, etc. It also is desirable that the solutions of precious metal catalysts be of high purity and essentially free of negative ions which may inhibit certain catalytic reactions.

U.S. Patent 3,318,891 (Hausman et al) describes a procedure for preparing crystalline palladium (II) acetate having utility as a catalyst. The palladium diacetate prepared in accordance with the process of the '891 patent is reported to be of high purity and is soluble in benzene. Crystalline complexes with benzene can be recovered from the benzene solutions. In the process, hydrated palladium oxide is first prepared by treatment of an aqueous solution of a soluble palladium salt with an alkaline reagent to precipitate the hydrated palladium oxide. The hydrated palladium oxide then is converted to palladium diacetate crystal.

U.S. Patent 3,700,458 (Lindholm) describes a chemical process for preparing noble metal salts of carboxylic acids useful in photosensitive and thermosensitive compositions. The process involves mixing a non-aqueous solution of an organic carboxylic acid with a non-aqueous solution of a noble metal trifluoroacetate or tetrafluoroborate in the presence of an organic pep tizer. A variety of organic peptizers are disclosed including polyvinyl acetals and certain acrylate copolymers.

US Patent 4,465,635 (Chang et al) describes a process for preparing palladous carboxylates by reaction of palladium metal with a carboxylic acid containing a hydrocarbon or a halogenated hydrocarbon in the presence of a member selected from the group consisting of nitric acid and nitrous oxide; $HNO_2$; nitric oxide and oxygen; nitric oxide and nitrogen dioxide; nitrogen dioxide; and nitrosyl acetate. The carboxylic acids utilized in the reaction contain from 1 to 10 carbon atoms.

In accordance with the present invention, a process for preparing a solution of a precious metal salt of an organic carboxylic acid is provided which comprises reacting the organic carboxylic acid or a mixture of such acids with a hydrated oxide of the precious metal, or a mixture of such oxides, in the presence of an organic liquid which is a solvent both for the carboxylic acid and the resulting precious metal salt.

In one particular procedure the hydrated precious metal oxide is prepared by dissolving the precious metal in aqua regia, and thereafter precipitating a hydrated precious metal oxide from the solution by adding an inorganic base, preferably an aqueous alkaline solution.

The process of the present invention results in the formation of solutions of precious metal carboxylate salts of high purity. These solutions are themselves useful as precious metal catalysts, as well as providing a source for the salts themselves also useful as precious metal catalysts.

The process of the invention is particularly useful for preparing palladium salts, salt solutions and palladium catalysts, and in the following discussion and examples much of it is specific to palladium, although it is to be understood that the process of the invention is equally applicable to salts of other precious metals such as silver, platinum, iridium, gold, rhodium and ruthenium, so that references to such other precious metals may generally be substituted for palladium in the following discussion.

One presently preferred method of the invention comprises the steps of:

(a) preparing a mixture comprising hydrated precious metal oxide, at least one organic carboxylic acid, and a hydrocarbon solvent;

(b) maintaining the mixture at a temperature of from about 25° to about 100° C for a period of time sufficient to form the desired precious metal salt; and

(c) recovering the desired precious metal salt as a hydrocarbon solution.

Hydrated palladium oxide can be prepared in accordance with any one of a variety of methods described in the prior art, but as already indicated, the preferred procedure involves dissolving palladium metal in aqua regia or nitric acid to form a solution, and thereafter precipitating hydrated palladium oxide from the solution by adding an inorganic base or aqueous alkaline solution. Alternatively, an inorganic palladium salt such as $PdCl_2$, $Pd(NO_3)_2$, $Na_2PdCl_4$, etc., can be dissolved in aqueous acid, and hydrated palladium oxide precipitated from the solution by adding a base either as a solid or as an aqueous solution. The amount of base or aqueous alkaline solution added is an amount sufficient to form and precipitate hydrated palladium oxide and to neutralize unreacted aqua regia and other inorganic acids present in the solution. Generally the amount of base or alkaline solution added will be sufficient to raise the pH of the mixture to about 8-10. Additional base can be added but may be difficult to wash out. The rate of addition should be controlled to prevent an excessive rise in temperature due to the heat of neutralization. Various conventional aqueous alkaline solutions can be utilized to effect the desired precipitation and neutralization, and aqueous alkali metal solutions are preferred. Examples include aqueous solutions containing sodium or potassium hydroxide, sodium carbonate, sodium bicarbonate, potassium carbonate, etc.

The precipitated hydrated palladium oxide prepared in the above manner is filtered and washed with water to remove salts. Care must be taken to avoid removing the water of hydration.

As noted, the preparation of the precious metal carboxylates by the process of the present invention is conducted in an organic solvent which can be any organic liquid in which the acid and the salt are soluble. Examples of organic solvents which can be utilized in the process of the present invention include hydrocarbon solvents which are capable of solvating the palladium carboxylate products. For this reason, aromatic solvents are preferred. Useful aromatic solvents include benzene, toluene, xylene, cumene, pseudo cumene and mesitylene. Aliphatic solvents include n-hexane and cyclohexane. Other organic solvents which can be used include mineral spirits, cellosolves and ketones such as acetone. The hydrocarbon solvents are particularly useful, and the most useful hydrocarbon solvents appear to be the aromatic hydrocarbon solvents. In some instances, other liquids such as alcohols may be useful as solvents.

The organic carboxylic acids from which the precious metal salts can be prepared include aliphatic, cycloaliphatic and aromatic mono- and polybasic carboxylic acids. The organic carboxylic acids may be either natural or synthetic, or mixtures thereof. Examples of natural acids, although usually refined, include straight and branched chain carboxylic acids and mixtures such as tall oil acids and cyclic carboxylic acids such as naphthenic acids. A variety of synthetic carboxylic acids, and particularly aliphatic carboxylic acids or mixtures thereof is useful, and these generally will contain six or more carbon atoms. The aliphatic carboxylic acids used in the present invention can contain from 2 to about 30 carbon atoms, and the alicyclic carboxylic acids can contain from 5 to about 30 carbon atoms. Aromatic carboxylic acids contain from 7 to about 30 carbon atoms. Generally the aliphatic carboxylic acids will contain from about 6 to about 30 carbon atoms and more preferably from about 6 to about 18 carbon atoms. When more than one carboxylic acid is employed, the carboxylic acids containing as few as two carbon atoms may be employed advantageously as one of the acids of the mixture. Examples of useful organic carboxylic acids include acetic acid, propionic acid, butyric acid, isopentanoic acid, hexanoic acid, 2-ethyl butyric acid, nonanoic acid, decanoic acid, 2-ethyl hexanoic acid, isooctanoic acid, isononanoic acid, neodecanoic acid, dodecanoic acid, lauric acid, palmitic acid, stearic acid, oleic acid, linoleic acid, commercially available mixtures of two or more carboxylic acids such as naphthenic acid, tall oil acids, rosin acids, benzoic acid, etc.

The reaction between the hydrated precious metal oxide and the organic carboxylic acid can be carried out at temperatures of from about 25° C to about 100° C for a period of time sufficient to form the desired precious metal salt. More often, the reaction temperature will be between about 50° and 75° C, and a temperature of about 60° C presently is preferred. Generally, the reaction will be completed in from about 0.5 hour to about 24 hours, and the period of time required for reacting the hydrated precious metal oxide with any particular carboxylic acid in solution can be readily determined by one skilled in the art.

An aqueous layer may be observed after the reaction is completed. The aqueous layer is removed, and the organic layer generally is filtered to remove any undesirable solids which may be present. The filtrate is a solution containing the desired precious metal salt. Depending on the amount of solvent used in the reaction, the filtrate may be concentrated under vacuum to provide solutions having higher concentrations of the desired precious metal salt.

In another embodiment of the present invention, the precious metal carboxylate is prepared by the steps of

(a) dissolving a precious metal salt from the group consisting of halides, nitrate, and sulfate in water to form an aqueous solution,

(b) adding an organic carboxylic acid and a solvent to the solution prepared in step (a) whereby a mixture of an organic phase and an aqueous phase is obtained,

(c) adding and mixing an inorganic base or an aqueous inorganic alkaline solution to the mixture obtained in step (b),

(d) allowing the mixture obtained in step (c) to separate into an aqueous phase and an organic phase, and

(e) recovering the organic phase containing the desired precious metal salt.

The solvent preferably is a hydrocarbon solvent and most preferably an aromatic hydrocarbon solvent as described above. The aqueous inorganic alkaline solution generally is an aqueous solution of sodium hydroxide, potassium hydroxide, sodium carbonate, etc., as described above. The inorganic base added as a solid may be any of the inorganic bases used in the alkaline solutions.

The concentration of the precious metal salt in the aqueous solution prepared in step (a) is not critical and may be varied over a wide range. Generally, the concentration of salt will be from about 5 to about 20% by weight. Similarly, the amount of organic carboxylic acid and hydrocarbon solvent added to the solution in step (b) is not critical. Typically, the reactants are mixed in about stoichiometric concentrations, but in some instances, an excess of a reactant may be used to force the reaction to completion. Generally, the amount of carboxylic acid will be about one equivalent of acid per equivalent of precious metal salt. The amount of solvent added to the mixture in step (b) should be an amount which will yield a solution of the desired precious metal carboxylate in acceptable concentration. Generally, the solutions of the salts of organic carboxylic acids obtained in accordance with the present invention preferably contain from about 3 to about 10 or 15% by weight of precious metal although metal concentrations as high as 30% or more can be obtained. The amount of inorganic base or aqueous inorganic alkaline solution added to the mixture in step (c) generally is an amount which will result in the conversion of the inorganic precious metal salt to a metal form which can react with the organic carboxylic acid and to neutralize any undesirable inorganic acids formed during the reaction or remaining after the reaction. Sufficient inorganic base or alkaline solution is added to raise the pH of the mixture to about 8 to 10. In one embodiment, sufficient aqueous alkaline solution is added to the mixture to result in a clear aqueous layer when the mixture is allowed to separate into an aqueous phase and an organic phase in step (d). If the aqueous phase is not clear, additional alkaline solution is added and the two phases are mixed by, for example, stirring, and thereafter allowed to separate. The organic phase is recovered and contains the desired precious metal salt. The organic phase can be further diluted with solvent or concentrated under vacuum to provide solutions containing the desired concentration of precious metal salt. The solutions may be filtered to remove suspended particles or washed with water to remove undesirable impurities.

The following examples illustrate the process of the present invention and palladium salt solutions which are prepared in accordance with the process of the invention. Unless otherwise indicated in the following examples and elsewhere in the specification and claims, all parts and percentages are by weight, all temperatures are in degrees celsius, and all pressures are at or near atmospheric.

Example 1

Palladium sponge (5 grams) is dissolved in 40 grams of aqua regia. To this solution there is added 40 grams of sodium hydroxide dissolved in 200 ml. of water. This mixture is stirred for one hour and filtered. The residue is washed with 400 ml. of water and recovered. The recovered residue is mixed with 15 grams of 2-ethyl-hexanoic acid dissolved in 200 ml. of xylene and heated to about 60°C whereupon most of the solid material dissolves in the xylene. The xylene solution is filtered and the remaining solids are washed with 100 ml. of xylene. The filtrate is the desired solution and is concentrated under vacuum to 60 grams. This solution contains about 5.9% palladium.

Example 2

Palladium sponge (31.1 grams) is dissolved in 100 ml. of aqua regia and thereafter evaporated to dryness. The solid is dissolved in 200 ml. of water, and to this solution 500 ml. of xylene and 110 grams of neodecanoic acid were added with stirring. To this mixture there is added 32 grams of sodium hydroxide in one gram portions with stirring until the aqueous layer is clear. The mixture is diluted with an additional 300 ml. of xylene, and the xylene layer is recovered by decantation and filtered through a filter aid. The filtrate is the desired solution of the palladium salt containing about 3.94% palladium.

Example 3

A palladium chloride solution containing 5 grams of palladium is prepared by dissolving palladium sponge in aqua regia, evaporating the mixture to dryness and dissolving the residue in water. Hydrated palladium oxide is prepared as in Example 2 except that the mixture is centrifuged to separate the hydrated palladium oxide and reslurried in distilled water to wash the solids. After centrifuging again, the hydrated palladium oxide is reslurried in 100 ml. of water and mixed with 30 grams of distilled naphthenic acid (193.3 acid value) in 100 ml. of xylene. The solution is warmed to 50° C and stirred vigorously. The layers are separated and a small residue is extracted with 50 ml. of xylene. The combined xylene layers are washed with 100 ml. of warm (50° C) water, and the xylene layer is filtered. The filtrate is the desired solution of the palladium salt of naphthenic acid.

Example 4

A hydrated palladium oxide containing 5 grams of palladium is prepared as described in Example 3, rinsed with 50 ml. of acetone and resuspended in 100 ml. of acetone after centrifuging. The acetone slurry is stirred at 60° C with 15.2 grams of benzoic acid in 50 ml. of acetone. After three hours, the solution is filtered, and the filtrate is the desired product.

Example 5

A hydrated palladium oxide containing 5 grams of palladium is prepared as described in Example 3, reslurried with 100 ml. of water and warmed with 27 grams of oleic acid in 100 ml. of xylene. The xylene layer is dried with 50 grams of anhydrous sodium sulfate, filtered, and the solids are washed with 100 ml. of xylene. The xylene solutions are combined and are the desired product.

The stability of the solutions prepared in accordance with this invention can be improved by incorporating various solubilizing and stabilizing agents such as, for example, ammonia, amines, chelating agents, etc.

The organic solutions, and in particular, the hydrocarbon solutions of the precious metal salts of carboxylic acids prepared in accordance with the present invention are useful as catalysts for hydrogenation reactions as is well known in the art. The solutions are particularly useful as a catalyst or as a catalyst component in homogeneously catalyzed organic reactions.

The precious metal salts of carboxylic acids prepared in accordance with the present invention can be recovered and isolated as solids from the solutions of the present invention by techniques well known in the art such as by precipitation, evaporation, etc. The solid precious metal salts can be decomposed under either oxidizing or reducing conditions to form the corresponding precious metal carboxylates or precious metal catalysts. For example, precious metal oxides can be thermally decomposed in the presence of oxygen at temperatures of about 350° C to form precious metal oxide catalysts, and the catalyst particles are characterized as having an enhanced surface area, particularly when compared to, for example, the surface area of particles obtained by the oxidative decomposition of palladium chloride at the same temperature.

The precious metal salts of the present invention which may be recovered from the solutions may be reduced under reducing conditions at elevated tempera tures (generally below 300° C) to form the desired precious metal catalysts. For example, palladium naphthenate isolated from a solution prepared in accordance with the present invention can be reduced to palladium metal at 170° C, and palladium neodecanoate isolated from solutions prepared in accordance with the present invention can be decomposed in a reducing atmosphere to palladium at a temperature of about 220° C. In contrast, palladium chloride cannot be reduced at a temperature of as high as 350° C. The metal or metal oxide catalyst prepared and isolated as described also is characterised as comprising small particle clusters which appear to be in the range of from about 5 to about 15 Angstroms or less.

Supported catalysts also can be prepared in accordance with this invention by (1) depositing the precious metal salt solutions of the invention on supports such as alumina, (2) drying the treated support to remove the solvent, and (3) decomposing the salt under either oxidizing or reducing conditions as described above to deposit the desired precious metal oxide or the precious metal on the support.

The precious metal carboxylate salts, salt solutions, and the precious metal and precious metal oxides which can be prepared from the solutions in accordance with the present invention also are characterised as being substantially free of chloride, nitrate and other detrimental anions which are known to reduce the effectiveness of these materials as catalysts.

While the invention has been explained in relation to its preferred embodiments, it is to be understood that various modifications thereof will become apparent to those skilled in the art upon reading the specification, and may be made without departing from the scope of the invention as herein described.

## Claims

1. A process for the preparation of organic carboxylic acid salts of precious metals characterised by the step of reacting the carboxylic acid, or a mixture of carboxylic acids, with a hydrated oxide of the precious metal, or mixture of such precious metal oxides, in the presence of an organic liquid which is a solvent for both the carboxylic acid reactant and the precious metal salt.

2. A process according to claim 1, characterised in that the carboxylic acid and the oxide are reacted at a temperature in the range 25 to 100°C.

3. A process according to claim 1 or 2, characterised in that the carboxylic acid reactant comprises one or more aliphatic carboxylic acids containing from 2 to 30 carbon atoms, and/or one or more alicyclic carboxylic acids containing from 5 to 30 carbon atoms, and/or one or more aromatic carboxylic acids containing from 7 to 30 carbon atoms.

4. A process according to claim 3, characterised in that the carboxylic acid reactant is an aliphatic or alicyclic carboxylic acid containing from 6 to 18 carbon atoms.

5. A process according to any one of claims 1 to 4, characterised in that the precious metal oxide is palladium oxide.

6. A process according to any one of claims 1 to 5, characterised in that the solvent is a liquid hydrocarbon.

7. A process according to claim 6, characterised in that the solvent is an aromatic hydrocarbon.

8. A process according to any one of claims 1 to 7, characterised in that the precious metal oxide reactant is prepared by the initial steps of:

(a) dissolving she precious metal in aqua regia or nitric acid to form a solution; and

(b) precipitating the hydrated precious metal oxide from the solution by adding an inorganic base or aqueous alkaline solution to the solution obtained in step (a).

9. A process according to claim 8, characterised in that the amount of aquous alkaline solution added in step (b) is sufficient to precipitate said hydrated precious metal oxide and also to neutralize unreacted aqua regia and other inorganic acids present.

10. A process according to claim 9, characterised in that, in step (b), sufficient base or aqueous alkaline solution is added to raise the pH of the mixture to a value of from 8 to 10.

11. A process according to any one of claims 1 to 9, characterised in that the hydrated precious metal oxide is formed in situ in the reaction medium.

12. A process according to claim 11, characterised in that the hydrated precious metal oxide is prepared by:

(a) dissolving a halide nitrate or sulfate salt of the precious metal in water to form an aqueous precious metal salt solution;

(b) adding the organic carboxylic acid reagent and the organic solvent, being a water immiscible solvent, to the solution prepared in step (a) thereby to form a mixture comprising an organic phase and an aqueous phase;

(c) adding and mixing an inorganic base or an aqueous inorganic alkaline solution to the mixture obtained in step (b);

(d) allowing the mixture obtained in step (c) to separate into an aqueous phase an an organic phase; and

(e) separating the organic phase containing the product precious metal carboxylate salt in solution.

13. A process according to any one of claims 1 to 12, characterised in that following the reaction of the carboxylic acid and the precious metal oxide, the product precious metal carboxylate is recovered from solution in the organic solvent.

14. A process according to claim 13, characterised in that following the recovery of the precious metal carboxylate from the solution, the salt is decomposed in the presence of oxygen at an elevated temperature to give a precious metal oxide.

15. A process according to claim 14, characterised in that the precious metal oxide is formed by decomposing the precious metal carboxylate salt at a temperature in the range 300 to 375°C.

16. A process according to claim 13, characterised in that following the recovery of the precious metal carboxylate from solution, the salt is decomposed under reducing conditions to give the precious metal in elemental form.

17. A process according to claim 16, characterised in that the elemental precious metal is formed by decomposing the precious metal carboxylate salt in a reducing atmosphere at a temperature of below about 300°C.

18. A precious metal-containing catalyst composition consisting of or comprising:

(a) a precious metal carboxylate salt solution obtained by a process as claimed in any one of claims 1 to 12;

(b) a precious metal carboxylate salt obtained by a process as claimed in claim 13;

(c) a precious metal oxide obtained by a process according to claim 14 or 15;

(d) an elemental precious metal obtained by a process according to claim 16 or 17.

19. A process for preparing a precious metal-containing catalyst according to claim 18(b), (c) or (d), which comprises impregnating a catalyst support material with a precious metal carboxylate salt solution obtained by a process as claimed in any one of claims 1 to 12, removing the solvent to deposit the carboxylate salt on the support and, optionally, oxidising or reducing the deposited salt in situ.